# EUROPEAN PATENT SPECIFICATION

(11) **EP 3 118 623 B1**
(45) Date of publication and mention of the grant of the patent: **27.05.2020**
(21) Application number: 16179753.5
(22) Date of filing: 15.07.2016
(51) Int. Cl.: G01N 33/50, G01N 33/569

(54) **METHOD AND DEVICE FOR STABILIZING OF PROTEINS**
VERFAHREN UND VORRICHTUNG ZUR STABILISIERUNG VON PROTEINEN
PROCÉDÉ ET DISPOSITIF DE STABILISATION DE PROTÉINES

(30) Priority: 15.07.2015 US 201562192645 P; 14.07.2016 US 201615209855
(43) Date of publication of application: 18.01.2017
(73) Proprietor: Streck Inc., La Vista, NE 68128 (US)
(72) Inventor: ALT, Jodi, Elkhorn, NE 68022 (US); MOORE, Carissa, Omaha, NE 68136 (US); HUNSLEY, Bradford, Papillion, NE 68133 (US)
(74) Representative: Bawden, Peter Charles

(56) References cited:
- EP-A1- 1 217 372
- WO-A2-03/018757
- US-A1- 2010 167 271
- Freda Passam ET AL: "Feasibility of an easily applicable method of ZAP-70 measurement in chronic lymphocytic leukemia in the routine flow cytometry setting: a methodological approach", ANNALS OF HEMATOLOGY, vol. 85, no. 11, 27 July 2006 (2006-07-27) , pages 795-805, XP055469729, DE ISSN: 0939-5555, DOI: 10.1007/s00277-006-0159-4

## Description

### FIELD OF THE INVENTION

This invention relates to an improved method for preparation of a biological sample for analysis of protein epitopes by flow cytometry.

### BACKGROUND

Flow cytometry is a technology used to analyze the characteristics of cells. Preparation of biological samples for analysis by flow cytometry often involves the fixation of cells. Flow cytometry may be utilized in cell counting, cell sorting, biomarker detection and protein engineering. Flow cytometry may be used in the diagnosis of various diseases such as cancer.

Cell surface markers or intracellular markers may be detected with flow cytometry. Flow cytometry uses fluorophore-conjugated antibodies that recognize specific epitopes on proteins to differentiate types of cells. Analysis of intracellular markers involves staining protocols which require fixation of cells. Fixation of cells is typically achieved by formaldehyde or paraformaldehyde. See for example, US Publication 2015/0010923.

Clinical flow cytometry utilizes antibodies to recognize protein epitopes exposed on the surface of the cells through extracellular or surface staining. The antibodies are added to whole blood, allowed to bind, and then the excess unbound antibodies are removed by washing in a buffer. The red blood cells are removed via a chemical lysing step and the remaining white blood cells are then run on a flow cytometer. A fixation step is often added after the staining and lysing as this stabilizes the samples to be tested at a later time. Fixation of the cells is always after the extracellular staining is complete as it is well known that formaldehyde alters protein structure and disrupts some antibody epitopes. Staining after formaldehyde fixation is generally dimmer due to decreased epitope availability. Detection of intracellular proteins by flow cytometry through intracellular staining requires that the fluorophore-conjugated antibody pass through the cellular membrane in order to bind to the epitope on the protein of interest. An intact cellular membrane on a live cell will not allow this, therefore a permeabilization step is used to disrupt the cell membrane. Various types of detergents can be used to permeabilize the cell membrane. However, the use of detergents without prior fixation destroys the cell membrane and releases the intracellular proteins.

Some commercially available staining kits include IntraPrep™ Permeabilization Reagent [P/N IM2388] by Beckman Coulter (5-7% by weight formaldehyde), IC Fixation Buffer [FB001] by Invitrogen (1-5% by weight paraformaldehyde), PerFix-nc Buffer 1, Fixative Reagent [P/N B31167] by Beckman Coulter (less than 10% by weight formaldehyde), Flow Cytometry Fixation Buffer (1X) [FC004] by R&D Systems (formaldehyde), Intracellular Fixation Buffer [00-8222] by eBioscience (4% formaldehyde), Fixation and Permeabilization Solution [554722] by BD Biosciences (4.2% formaldehyde) and Lyse/Fix Buffer 5X [558049] by BD Biosciences (20.35% formaldehyde).

Although, methods for preparing biological samples for flow cytometry analysis have been employed reliably for many years, see for example U.S. Patent No. 6,977,156 and U.S. Patent No. 6,794,152 there remains a need for continued improvements. Biological samples, such as blood are subject to rapid deterioration. Formaldehyde is well known as a toxic substance. Some antigenic determinants can be sensitive to formaldehyde.

Certain protein epitopes have been found to be particularly challenging. Zap-70 is an intracellular kinase that is naturally expressed in T cells and NK cells and abnormally expressed in B cells in B-cell Chronic Lymphocytic Leukemia (B-CLL). Zap-70 analysis is used in the prognosis and monitoring of B-cell Chronic Lymphocytic Leukemia (B-CLL). The amount of Zap-70 expressed in B-CLL is correlated with survival. However, Zap-70 expression is not stable in drawn blood. Zap-70 expression may decrease by 10% per day in blood collection tubes. There is no consensus among clinical laboratories for the type of blood collection tube used for the Zap-70 assay. Attempts to harmonize Zap-70 detection have suffered from the instability of Zap-70 expression and low population resolution due to dim staining.

Accordingly, there is a need for an improved method of preserving a biological sample for analysis by flow cytometry. There is a need for a method which provides increased stability of samples both before and after staining protocols. There is a need for a method which allows for samples which are stable for shipping. There is a need for a method which utilizes a relatively non-toxic preservative. There is a need for a method which does not interfere with the analysis of certain cell characteristics. There is a need for a method which provides increased stability of cell surface markers or intracellular markers for detection by flow cytometry. There is a need for clinically relevant assays of protein epitopes, such as Zap-70.

The present invention addresses one or more of the above needs by providing a method of contacting a drawn blood sample with a protective agent that stabilizes the sample prior to any staining process but does not compromise flow cytometry test results and analyzing the white blood cells of the contacted sample for protein epitopes after at least about twenty-four hours after contacting.

### SUMMARY

The present teachings contemplate a method comprising contacting a drawn blood sample with a protective agent that is substantially free of formaldehyde and analyzing the white blood cells of the contacted sample for protein epitopes after at least about twenty-four hours after contacting.

The contacted blood sample may be stable for analysis for at least four days at about 22°C. The contacted blood sample may be stable for analysis for at least seven days at about 22°C.

The protective agent may be present in the amount of from about 25 µl to about 125 µl. The protective agent may be present in the amount of about 25 µl to about 125 µl per 5ml of liquid biological sample. The protective agent may be present in the amount of about 75 µl per 5ml of liquid biological sample. The protective agent may include a preserving agent and an anticoagulant. The preserving agent may be selected from aldehyde, oxazolidine, alcohol, cyclic urea, and mixtures thereof. The preserving agent may be imidazolidinyl urea or diazolidinyl urea. The anticoagulant may be K3EDTA.

The contacted blood sample may be analyzed for protein epitopes of intracellular markers. The contacted blood sample may be analyzed for protein epitopes of cell surface markers. The surface markers for analysis may be selected from the group consisting of: CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, CD200, FMC7, HLA-DR, Kappa, Lambda, TCR αβ, and TCR γδ. The intracellular markers for analysis may be selected from the group consisting of: cCD3, CD79a, cLambda, cKappa, TdT, BCL-2, and Zap70. The contacted blood sample may be analyzed for the expression of a protein tyrosine kinase.

The method may include the use of fluorophore conjugated antibodies. The method may include a step of intracellular staining. The method may be free of addition of formaldehyde or paraformaldehyde prior to or post a cellular staining process. The analysis may be accomplished by flow cytometry. The recoverable protein expression of the contacted blood sample after about four days may be about 90% of recoverable protein expression at the time the blood is drawn. The recoverable protein expression of the contacted blood sample after about four days may be about 100% of recoverable protein expression at the time the blood is drawn. The recoverable protein expression of the contacted blood sample stored at 22°C may occur within about 24 hours to about 96 hours after contact. The analysis may include screening for cancer.

The present teachings provide an improved method of preserving a biological sample for analysis by flow cytometry. The present teachings provide increased stability of samples. The present teachings provide samples which are stable for shipping. The present teachings provide a relatively non-toxic preservative. The present teachings provide a preservative that does not interfere with the analysis of certain cell characteristics. The present teachings provide increased stability of cell surface markers or intracellular markers for detection by flow cytometry. The present teachings provide a clinically relevant assay by the stabilization of Zap-70 in whole blood.

### BRIEF DESCRIPTION OF THE DRAWINGS

FIG. 1 depicts flow cytometry results of Protective Agent blood collection tube samples with and without a secondary fixation step.
FIG. 2 depicts data of Protective Agent blood collection tube samples stored at 22°C.
FIG. 3 depicts data of EDTA blood collection tube samples stored at 22°C and 6°C.
FIG. 4 depicts data of ACD Sol B blood collection tube samples stored at 22°C and 6°C.
FIG. 5 depicts data of Na Heparin blood collection tube samples stored at 22°C and 6°C.
FIG. 6 depicts a comparison of EDTA, ACD Sol B, Na Heparin, and Protective Agent blood collection tube samples from 2 hours to 96 hours.
FIG. 7 depicts the temperature variation of samples subjected to two day shipping.
FIG. 8 depicts a comparison of flow cytometry results of Zap-70 48 hours after shipping in Protective Agent blood collection tube and EDTA blood collection tube.

### DETAILED DESCRIPTION

The present invention is defined in the appended claims.

The explanations and illustrations presented herein are intended to acquaint others skilled in the art with the teachings, its principles, and its practical application. Those skilled in the art may adapt and apply the teachings in its numerous forms, as may be best suited to the requirements of a particular use. Accordingly, the specific embodiments of the present teachings as set forth are not intended as being exhaustive or limiting of the teachings. The scope of the teachings should, therefore, be determined not with reference to the above description, but should instead be determined with reference to the appended claims, along with the full scope of equivalents to which such claims are entitled.

This application claims the benefit of the filing date of U.S. Provisional Application No. 62/192,645, filed July 15, 2015.

The present teachings contemplate a method comprising contacting a drawn blood sample with a protective agent that is substantially free of formaldehyde and analyzing the white blood cells of the contacted sample for protein epitopes after at least about twenty-four hours after contacting. The protective agent may be included in a blood collection tube. The protective agent may be included as part of a kit.

The present teachings allow for increased antigen stability of intracellular markers such as Zap-70. Zap-70 is an intracellular kinase, whose level of aberrant expression in B-cell Chronic Lymphocytic Leukemia (B-CLL) is used for prognosis and treatment decisions. The varying expression, dim staining, and labile nature makes measuring Zap-70 challenging and has prevented it from becoming more widely used in Leukemia/ Lymphoma screening. The present teachings provide for stabilization of Zap-70, thereby allowing for a longer analysis window and more precise results. The present teachings provide increased fluorescence and population resolution of Zap-70. The present teachings allow for enhanced recovery of Zap-70 percentages up to 96 hours after blood draw.

The present teachings contemplate a method comprising contacting a drawn blood sample with a protective agent that is substantially free of formaldehyde and analyzing the white blood cells of the contacted sample for protein epitopes after at least about twenty-four hours after contacting. The protective agent may stabilize the sample prior to any staining process but does not compromise flow cytometry test results.

The contacted blood sample may be stable for analysis for at least four days at about 22°C. The contacted blood sample may be stable for analysis for at least seven days at about 22°C.

The protective agent may be present in the amount of about 75 µl. The protective agent may be present in the amount of about 75 µl per 5ml of liquid biological sample. The protective agent may be present in an amount of about 200 µl. The protective agent may be present in the amount of about 200 µl per 10 ml of liquid biological sample. The protective agent may be within a tube. The protective agent may be within in a blood collection tube. The tube containing the protective agent may receive about 2 ml to about 10 ml of patient blood. The tube containing the protective agent may receive about 2 ml of patient blood. The tube containing the protective agent may receive about 5 ml of patient blood. The tube containing the protective agent may receive about 10 ml of patient blood. The patient blood may be drawn directly into the tube containing the protective agent.

The protective agent may include a preserving agent and an anticoagulant. The preserving agent may be selected from aldehyde, oxazolidine, alcohol, cyclic urea, and mixtures thereof. The preserving agent may be a mixture of bicyclic oxazolidines. The preserving agent may be DMDM hydantoin. The preserving agent may be imidazolidinyl urea or diazolidinyl urea. The imidazolidinyl urea may be present in an amount of about 300 g/l to about 700 g/l of the protective agent. The imidazolidinyl urea may be about 1% to about 3% of the protective agent. The diazolidinyl urea may be present in an amount of about 125 g/l to about 525 g/l of the protective agent. The diazolidinyl urea may be about .05 % to about 2.5% of the protective agent. The anticoagulant may be K3EDTA. The K3EDTA may be present in an amount of about 60 g/l to about 100 g/l of the protective agent. The K3EDTA may be present in an amount of about 80 g/l to about 120 g/l of the protective agent. The protective agent may include a preserving agent, an anticoagulant and glycine. The glycine may be present in an amount of about 20 g/l to about 60 g/l of the protective agent. The protective agent may comprise an active agent in solution. Suitable solvents may include water, saline, dimethylsulfoxide, alcohol and mixtures thereof. The protective agent may comprise diazolidinyl urea (DU) and/or imidazolidinyl urea (IDU) in a buffered salt solution.

The contacted blood sample maybe analyzed for protein epitopes of intracellular markers. The contacted blood sample may be analyzed for protein epitopes of cell surface markers. The markers may be stable for at least 4 days in whole blood samples stored in a protective agent blood collection tube at 22°C (e.g., room temperature (free of any freezing step)). The markers may be stable for at least 7 days in whole blood samples stored in a protective agent blood collection tube at 22°C. The markers may be stable for about 4 to about 7 days in whole blood samples stored in a protective agent blood collection tube at 22°C.

The surface markers for analysis may be selected from the group consisting of: CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, FMC7, HLA-DR, Kappa, Lambda, TCR αβ, and TCR γδ. The intracellular markers for analysis may be selected from the group consisting of: cCD3, CD79a, cLambda, cKappa, TdT, BCL-2, and Zap70. The contacted blood sample may be analyzed for protein epitopes of the group consisting of: CD79a, TdT, and BCL-2. The contacted blood sample may be analyzed for the expression of a protein tyrosine kinase. The contacted blood sample may be analyzed for Zap 70 protein expression.

The method may include the use of fluorophore conjugated antibodies. The method may include a step of intracellular staining. The method may include intracellular staining without fixation of cells by formaldehyde or paraformaldehyde. The method may be free of addition of formaldehyde or paraformaldehyde prior to or post a cellular staining process. The analysis may be accomplished by flow cytometry. The recoverable protein expression of the contacted blood sample after about four days may be about 90% of recoverable protein expression at the time the blood is drawn. The recoverable protein expression of the contacted blood sample after about four days may be about 100% of recoverable protein expression at the time the blood is drawn. The recoverable protein expression of the contacted blood sample stored at 22°C may occur within about 24 hours to about 96 hours after contact. The analysis may include screening for cancer.

### EXAMPLE

Human blood samples were prepared for flow cytometry analysis. 50 µLs of whole blood drawn into the indicated blood collection tubes were stained with 5 µL of α-CD3-FITC, α-CD45-PerCP, and α-CD19-APC for 20 minutes at 22°C. The samples were fixed or not as indicated with a formaldehyde based commercial fixation reagent for 20 minutes at 22°C and then washed in PBS with BSA. The samples were permeabilized with a saponin based commercial perm/lyse reagent and stained with 20 µL of α-Zap-70-PE for 20 minutes at 22°C. The samples were washed twice in PBS with BSA and fixed in 1% paraformaldehyde in PBS for 30 minutes at 22°C before being run on a BD FACSCalibur. Percentages of T, NK, and B cells were obtained by the BD Multitest ™ HIV panel. All antibodies and blood collection tubes other than those containing the protective agent described herein were from BD Biosciences. Flow cytometry was performed on a BD FACSCalibur. External flow cytometry was performed by Flow Contract Site Laboratory on samples sent from Omaha, NE to Kirkland, WA and run on a BD FACSCanto. Data are representative from multiple experiments with multiple donors.

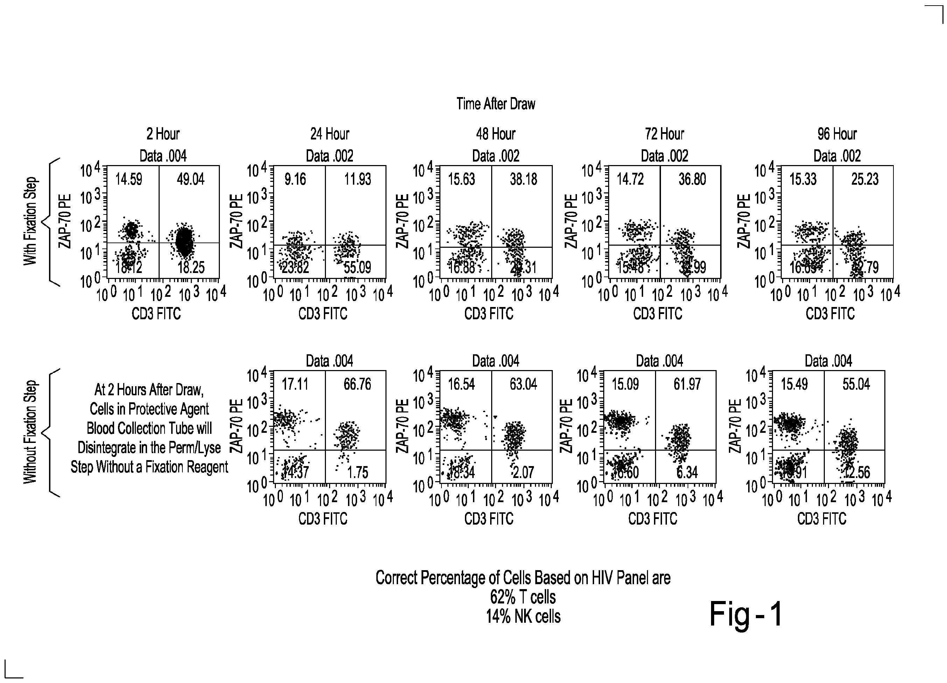

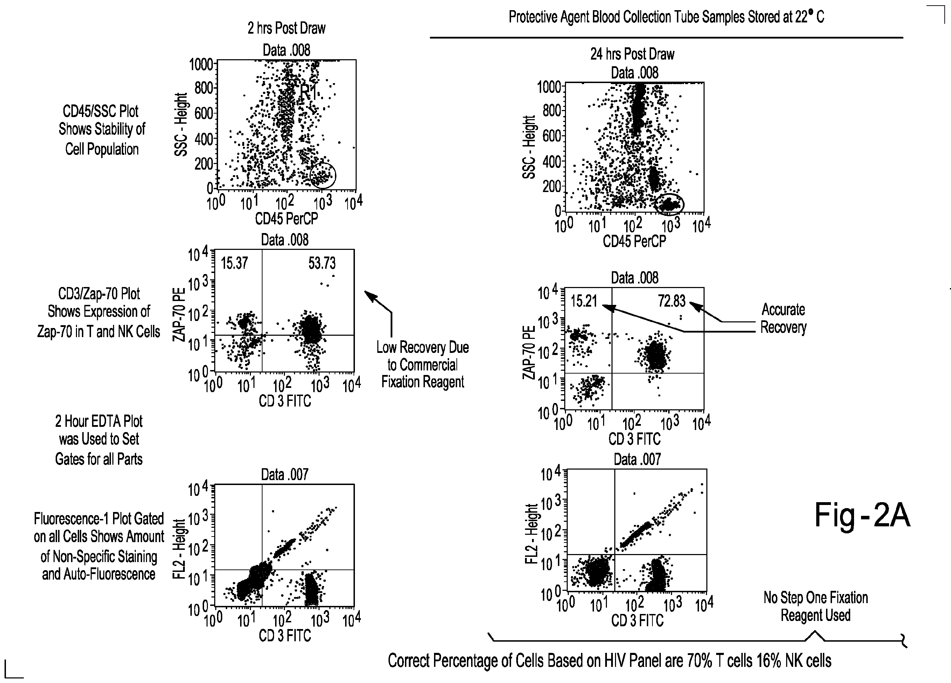

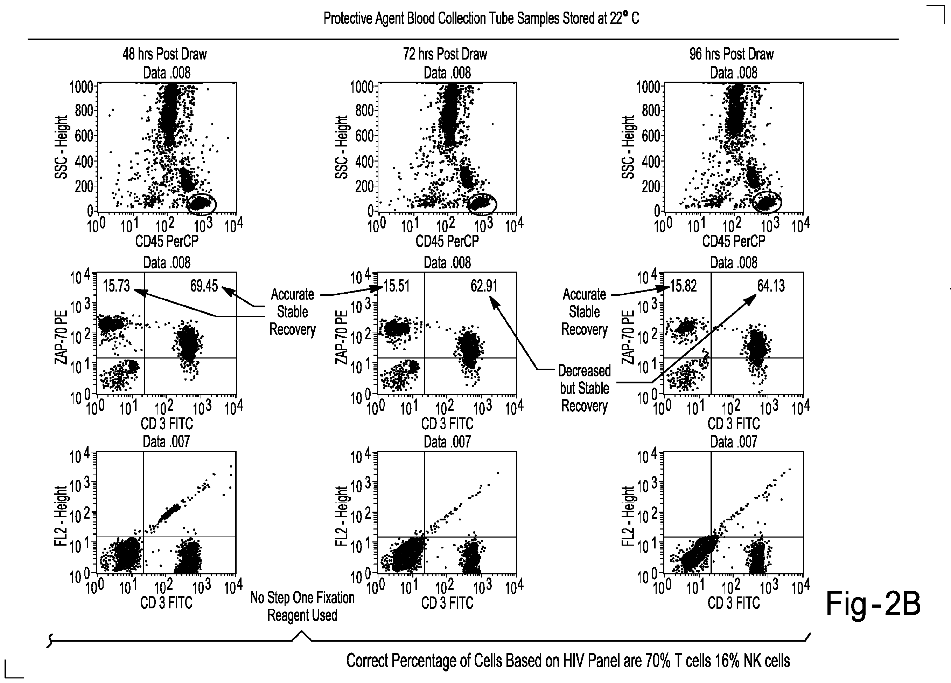

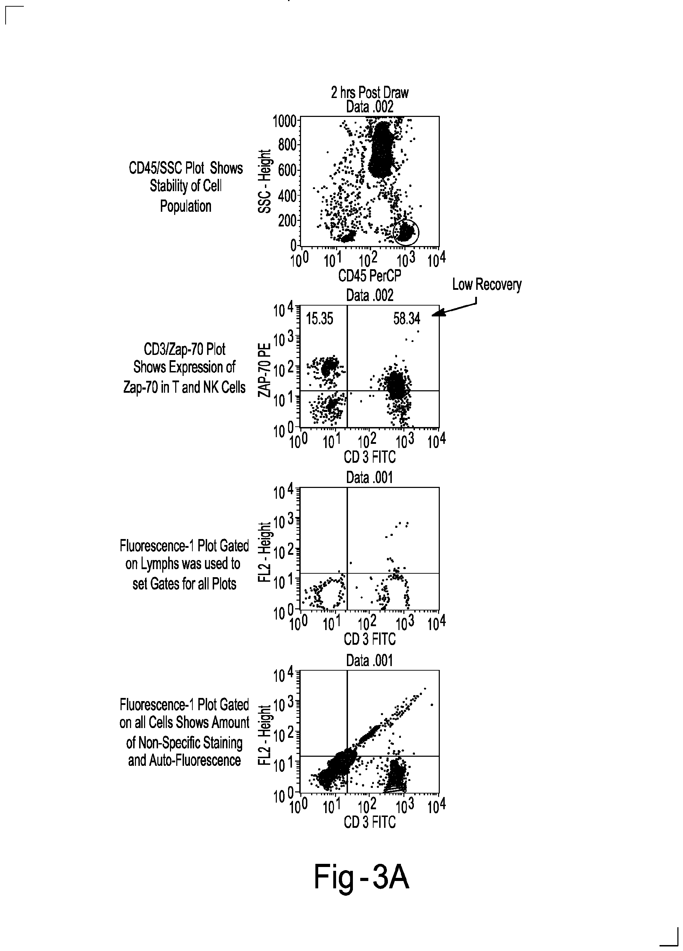

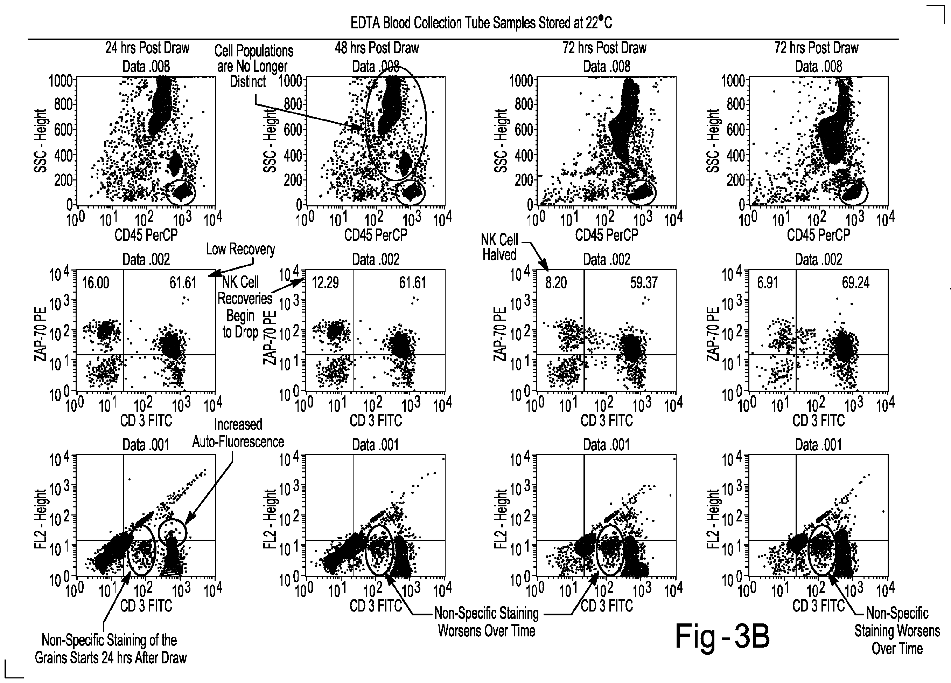

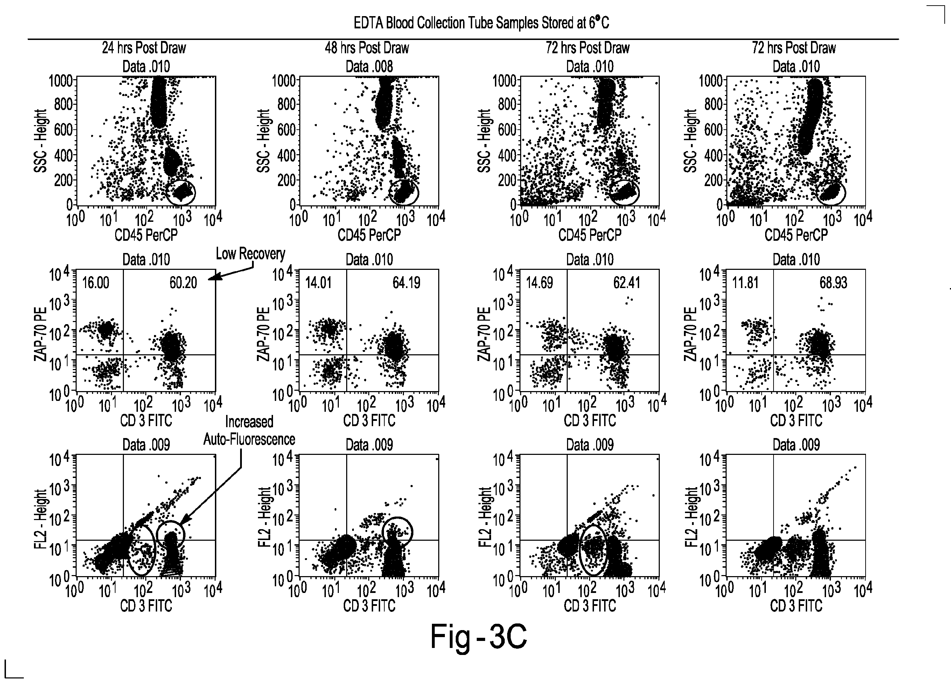

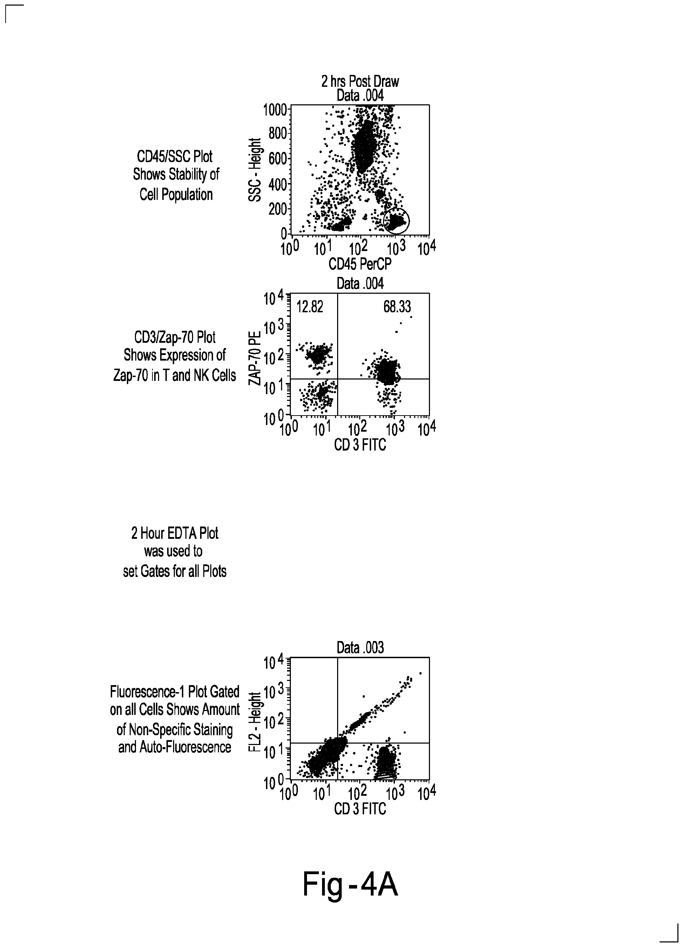

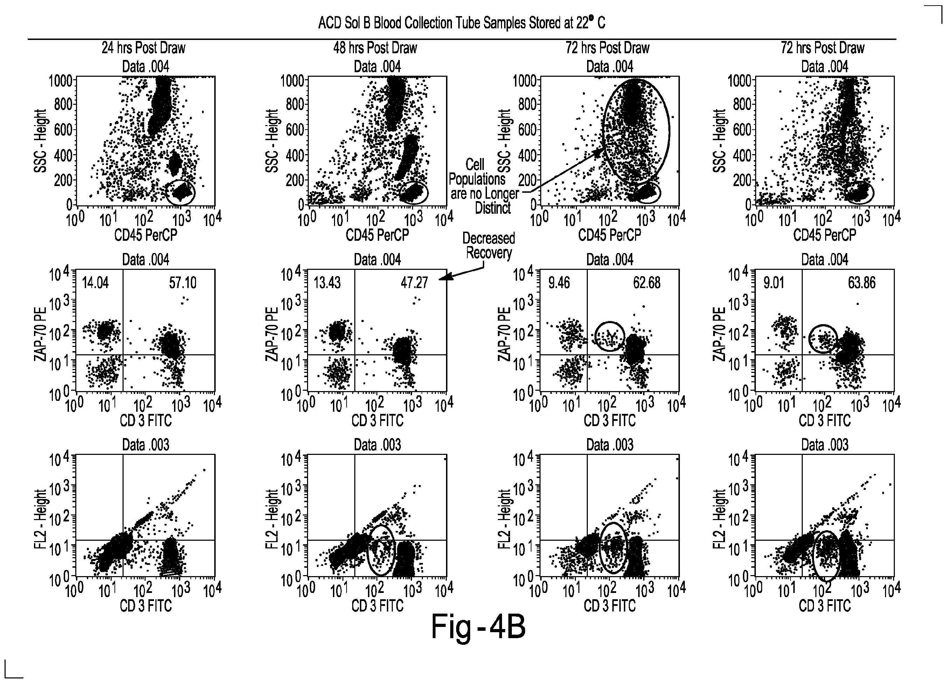

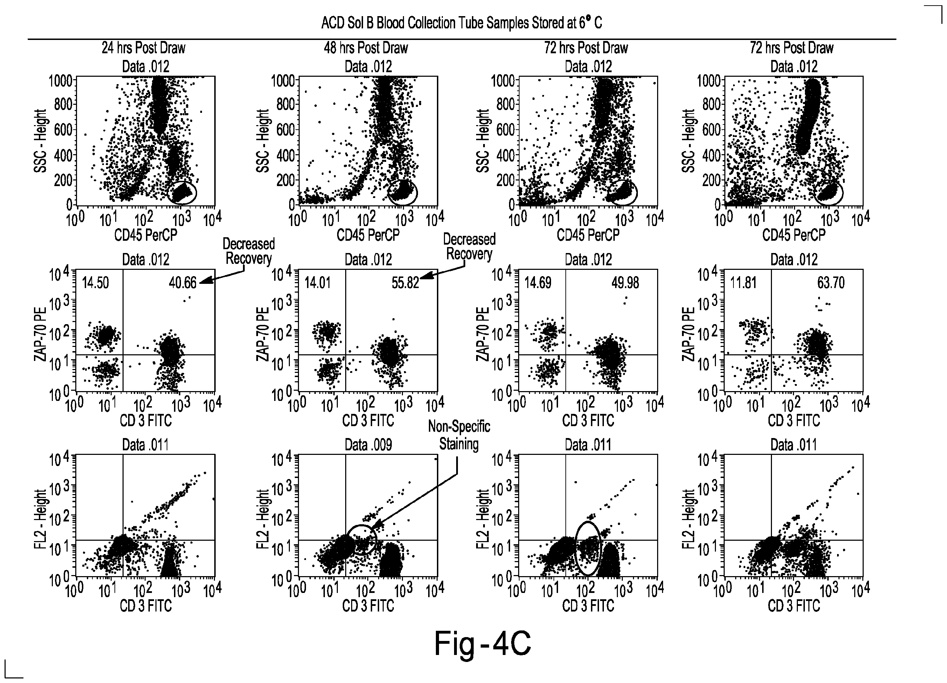

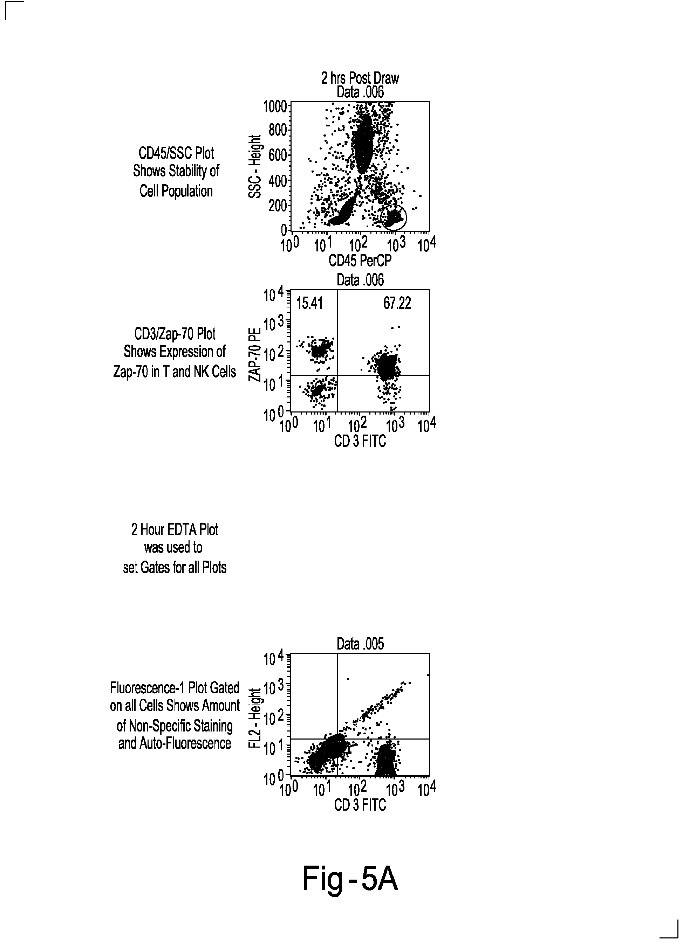

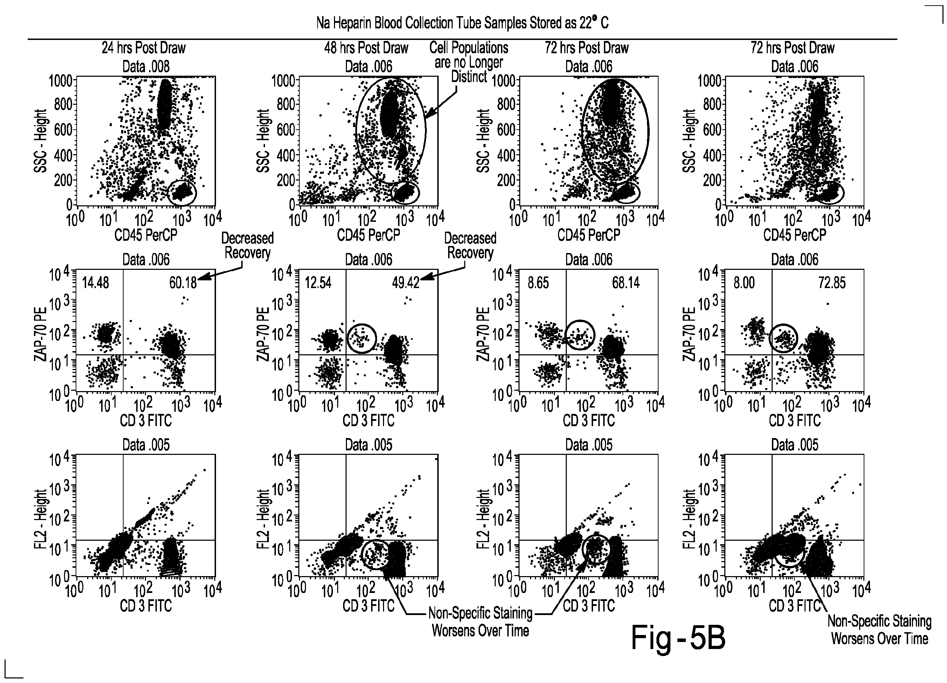

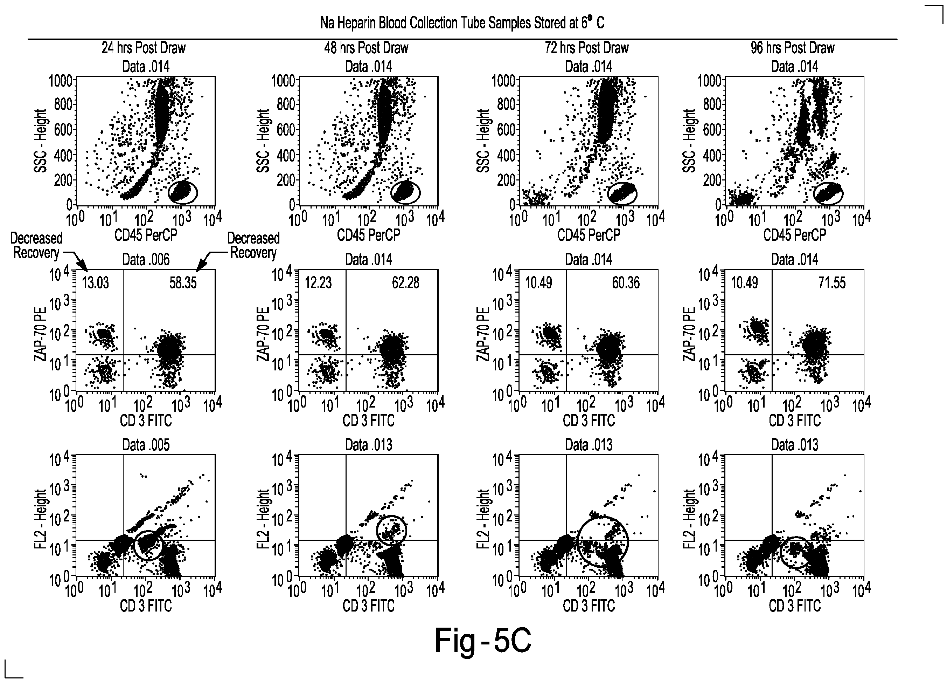

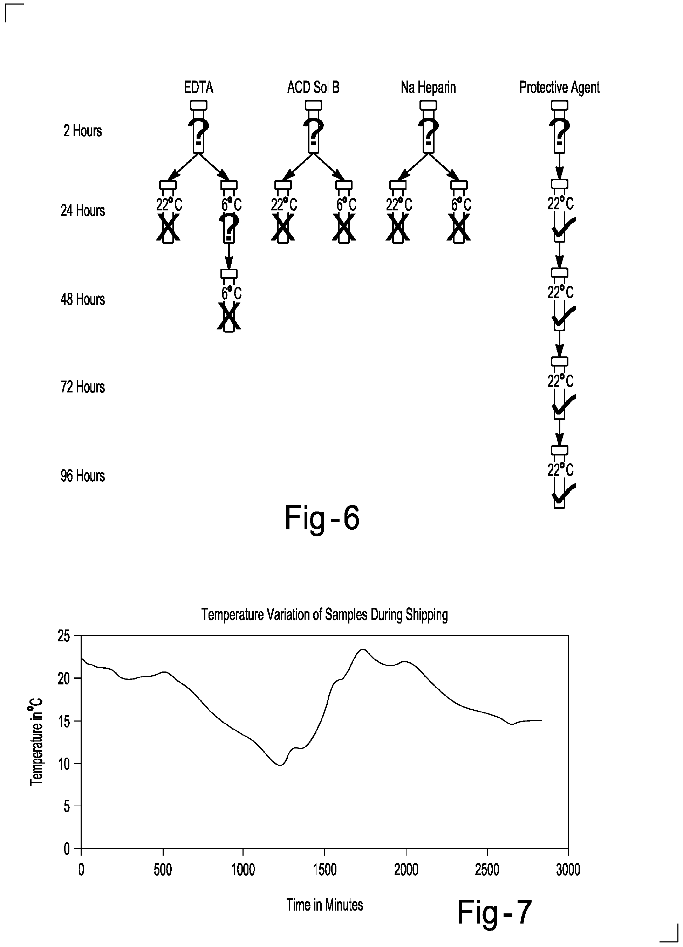

**FIG. 1** illustrates flow cytometry results of samples that underwent intracellular staining with and without a fixation step, demonstrating that the Protective Agent described herein allows for Zap-70 intracellular staining and analysis without fixation of cells. **FIG. 2** depicts data of Protective Agent blood collection tube samples stored at 22°C. **FIG. 3** depicts data of EDTA blood collection tube samples stored at 22°C and 6°C. **FIG. 4** depicts data of ACD Sol B blood collection tube samples stored at 22°C and 6°C. **FIG. 5** depicts data of Na Heparin blood collection tube samples stored at 22°C and 6°C. **FIGS. 2-5** illustrate that the Protective Agent blood collection tube provides superior stabilization of Zap-70 and population resolution in comparison to other blood collection tubes. The other tested blood collection tubes lost accurate recovery ≤24 hours. **FIG. 6** depicts a comparison of EDTA, ACD Sol B, Na Heparin, and Protective Agent blood collection tube samples from 2 hours to 96 hours. The findings indicate that the Protective Agent blood collection tube allows for accurate recovery of Zap-70 from blood samples stored at 22°C from 24 hours to 96 hours.

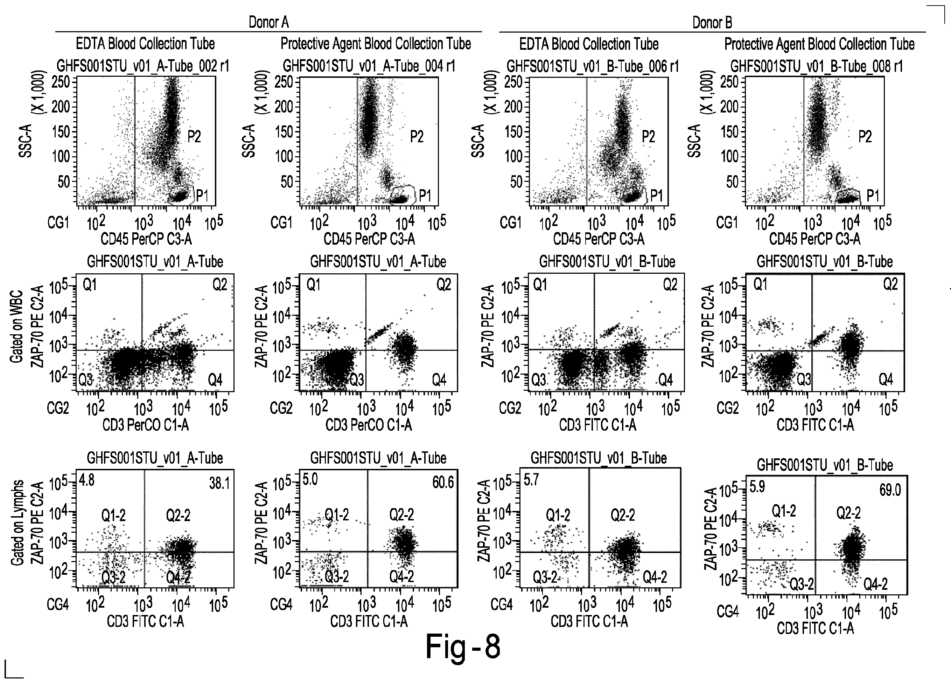

The shipped samples were shipped via two day shipping in order to examine the effects of temperature variation and stability on the shipped samples. **FIG. 7** illustrates the range of temperature variations the samples were subjected to in a 48 hour period. **FIG. 8** depicts a comparison of flow cytometry results of the shipped samples in Protective Agent blood collection and EDTA blood collection tube. **FIG. 8** illustrates Zap-70 is stable 48 hours after shipping in Protective Agent blood collection tube.

The present teachings provide stabilization of Zap-70 up to 96 hours after draw. The present teachings show increased Zap-70 fluorescence and population resolution after intracellular staining without formaldehyde or paraformaldehyde fixation using samples contacted with the protective agent of the present teachings. The protective agent may stabilize the sample prior to any staining process but does not compromise flow cytometry test results. The protective agent may allow for accurate flow cytometry results utilizing a staining process that is substantially free of any secondary fixation step (e.g., a formaldehyde fixation step).

Any numerical values recited herein include all values from the lower value to the upper value in increments of one unit provided that there is a separation of at least 2 units between any lower value and any higher value. As an example, if it is stated that the amount of a component or a value of a process variable such as, for example, temperature, pressure, time and the like is, for example, from 1 to 90, preferably from 20 to 80, more preferably from 30 to 70, it is intended that values such as 15 to 85, 22 to 68, 43 to 51, 30 to 32 etc. are expressly enumerated in this specification. For values which are less than one, one unit is considered to be 0.0001, 0.001, 0.01 or 0.1 as appropriate. These are only examples of what is specifically intended and all possible combinations of numerical values between the lowest value and the highest value enumerated are to be considered to be expressly stated in this application in a similar manner.

Unless otherwise stated, all ranges include both endpoints and all numbers between the endpoints. The use of "about" or "approximately" in connection with a range applies to both ends of the range. Thus, "about 20 to 30" is intended to cover "about 20 to about 30", inclusive of at least the specified endpoints.

The term "consisting essentially of" to describe a combination shall include the elements, ingredients, components or steps identified, and such other elements ingredients, components or steps that do not materially affect the basic and novel characteristics of the combination. The use of the terms "comprising" or "including" to describe combinations of elements, ingredients, components or steps herein also contemplates embodiments that consist essentially of or even consists of the elements, ingredients, components or steps. Plural elements, ingredients, components or steps can be provided by a single integrated element, ingredient, component or step. Alternatively, a single integrated element, ingredient, component or step might be divided into separate plural elements, ingredients, components or steps. The disclosure of "a" or "one" to describe an element, ingredient, component or step is not intended to foreclose additional elements, ingredients, components or steps.

It is understood that the above description is intended to be illustrative and not restrictive. Many embodiments as well as many applications besides the examples provided will be apparent to those of skill in the art upon reading the above description. The scope of the invention should, therefore, be determined not with reference to the above description, but should instead be determined with reference to the appended claims, along with the full scope of equivalents to which such claims are entitled. The omission in the following claims of any aspect of subject matter that is disclosed herein is not a disclaimer of such subject matter, nor should it be regarded that the inventors did not consider such subject matter to be part of the disclosed inventive subject matter.

## Claims

1. A method for analyzing white blood cells for a protein epitope of an intracellular marker comprising
contacting a drawn blood sample comprising the white blood cells with a protective agent that is substantially free of formaldehyde; wherein the protective agent comprises a preserving agent and an anticoagulant, wherein the preserving agent is imidazolidinyl urea or diazolidinyl urea and the anticoagulant is K₃EDTA, wherein the blood sample is stable for analysis for at least 4 days after contacting, and wherein the blood sample is stable at about 22° C after contacting.

2. The method of claim 1, wherein the protective agent is present in the amount of from 25 µl to 125 µl per 5 ml of the drawn sample.

3. The method of any of claim 1 or claim 2, which includes a step of intracellular staining.

4. The method of any of claims 1-3, wherein the intracellular marker is cCD3, CD79a, cLambda, cKappa, TdT, BCL-2 or Zap-70.

5. The method of any of claims 1-4, wherein the blood sample is stable for analysis of the intracellular marker for at least 7 days after the contacting.

6. The use of a protective agent comprising a preserving agent and an anticoagulant and is substantially free of formaldehyde for stabilizing a protein epitope of an intracellular marker in a sample of drawn blood wherein the preserving agent is imidazolidinyl urea or diazolidinyl urea and the anticoagulant is K3EDTA, wherein the blood sample is stable for analysis for at least 4 days after contacting, and wherein the blood sample is stable at about 22° C after contacting.

7. The use according to claim 6 wherein the intracellular marker is cCD3, CD79a, cLambda, cKappa, TdT, BCL-2 or Zap-70.

8. The use according to claim 6 or claim 7, to enable analysis of the intracellular marker at least 7 days after blood draw.

9. The method of any of claims 1-5, wherein analysis includes screening for cancer.

10. The method of any of claims 1-5 or 9, further comprising analyzing the blood sample for a protein epitope of a cell surface marker.

11. The method of claim 10, wherein the cell surface marker is CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, CD200, FMC7, HLA-DR, Kappa, Lambda, TCR αβ, or TCR γδ.

12. The use according to any of claims 6 to 8, wherein analysis includes screening for cancer.

13. The use according to any of claims 6 to 8 or 12, further comprising analyzing the blood sample for a protein epitope of a cell surface marker.

14. The use according to claim 13, wherein the cell surface marker is CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, CD200, FMC7, HLA-DR, Kappa, Lambda, TCR αβ, or TCR γδ.

## Patentansprüche

1. Verfahren zum Analysieren von weißen Blutkörperchen auf ein Epitop eines Proteins eines intrazellulären Markers, das umfasst:
Kontaktieren einer entnommenen Blutprobe, die die weißen Blutkörperchen enthält, mit einem Schutzmittel, das im Wesentlichen frei von Formaldehyd ist; wobei das Schutzmittel ein Konservierungsmittel und einen Gerinnungshemmer umfasst, wobei das Konservierungsmittel Imidazolidinylharnstoff oder Diazolidinylharnstoff und der Gerinnungshemmer K₃EDTA ist, wobei die Blutprobe für mindestens 4 Tage nach dem Kontaktieren stabil zur Analyse ist und wobei die Blutprobe nach dem Kontaktieren bei ungefähr 22°C stabil ist.

2. Verfahren gemäß Anspruch 1, wobei das Schutzmittel in einer Konzentration von 25 µl bis 125 µl pro 5 ml der entnommenen Probe vorliegt.

3. Verfahren gemäß einem der Ansprüche 1 oder 2, das umfasst: einen Schritt zum intrazellulären Färben.

4. Verfahren gemäß einem der Ansprüche 1 bis 3, wobei der intrazelluläre Marker cCD3, CD79a, cLambda, cKappa, TdT, BCL-2 oder Zap-70 ist.

5. Verfahren gemäß einem der Ansprüche 1 bis 4, wobei die Blutprobe für mindestens 7 Tage nach dem Kontaktieren stabil zur Analyse des intrazellulären Markers ist.

6. Verwendung eines Schutzmittels, das ein Konservierungsmittel und einen Gerinnungshemmer umfasst und im Wesentlichen frei von Formaldehyd ist, zum Stabilisieren eines Epitops eines Proteins eines intrazellulären Markers in einer entnommenen Blutprobe, wobei das Konservierungsmittel Imidazolidinylharnstoff oder Diazolidinylharnstoff und der Gerinnungshemmer K₃EDTA ist, wobei die Blutprobe für mindestens 4 Tage nach dem Kontaktieren stabil zur Analyse ist und die Blutprobe nach dem Kontaktieren bei ungefähr 22°C stabil ist.

7. Verwendung gemäß Anspruch 6, wobei der intrazelluläre Marker cCD3, CD79a, cLambda, cKappa, TdT, BCL-2 oder Zap-70 ist.

8. Verwendung gemäß Anspruch 6 oder Anspruch 7 zum Ermöglichen einer Analyse des intrazellulären Markers mindestens 7 Tage nach einer Blutentnahme.

9. Verfahren gemäß einem der Ansprüche 1 bis 5, wobei die Analyse eine Krebsvorsorgeuntersuchung umfasst.

10. Verfahren gemäß einem der Ansprüche 1 bis 5 oder 9, das weiterhin umfasst: Analysieren der Blutprobe auf ein Epitop eines Proteins eines Zelloberflächenmarkers.

11. Verfahren gemäß Anspruch 10, wobei der Zelloberflächenmarker CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, CD200, FMC7, HLA-DR, Kappa, Lambda, TCR αβ oder TCR γδ ist.

12. Verwendung gemäß einem der Ansprüche 6 bis 8, wobei die Analyse eine Krebsvorsorgeuntersuchung umfasst.

13. Verwendung gemäß einem der Ansprüche 6 bis 8 oder 12, die weiterhin umfasst: Analysieren der Blutprobe auf ein Epitop eines Proteins eines Zelloberflächenmarkers.

14. Verwendung gemäß Anspruch 13, wobei der Zelloberflächenmarker CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, CD200, FMC7, HLA-DR, Kappa, Lambda, TCR αβ oder TCR γδ ist.

## Revendications

1. Procédé d'analyse de globules blancs portant sur un épitope protéique d'un marqueur intracellulaire, comprenant :
la mise en contact d'un échantillon de sang prélevé comprenant les globules blancs avec un agent protecteur qui est sensiblement exempt de formaldéhyde ; dans lequel l'agent protecteur comprend un agent de conservation et un anticoagulant, dans lequel l'agent de conservation est l'imidazolidinylurée ou la diazolidinylurée et l'anticoagulant est le K₃EDTA, dans lequel l'échantillon de sang reste stable pour l'analyse pendant au moins 4 jours après la mise en contact, et dans lequel l'échantillon de sang reste stable à environ 22 °C après la mise en contact.

2. Procédé selon la revendication 1, dans lequel l'agent protecteur est présent dans une quantité de 25 µl à 125 µl par 5 ml de l'échantillon prélevé.

3. Procédé selon l'une quelconque de la revendication 1 ou de la revendication 2, lequel comprend une étape de coloration intracellulaire.

4. Procédé selon l'une quelconque des revendications 1 à 3, dans lequel le marqueur intracellulaire est cCD3, CD79a, cLambda, cKappa, TdT, BCL-2 ou Zap-70.

5. Procédé selon l'une quelconque des revendications 1 à 4, dans lequel l'échantillon de sang reste stable pour l'analyse du marqueur intracellulaire pendant au moins 7 jours après la mise en contact.

6. Utilisation d'un agent protecteur comprenant un agent de conservation et un anticoagulant et qui est sensiblement exempt de formaldéhyde pour stabiliser un épitope protéique d'un marqueur intracellulaire dans un échantillon de sang prélevé, dans laquelle l'agent de conservation est l'imidazolidinylurée ou la diazolidinylurée et l'anticoagulant est le K₃EDTA, dans laquelle l'échantillon de sang reste stable pour l'analyse pendant au moins 4 jours après la mise en contact, et dans laquelle l'échantillon de sang reste stable à environ 22 °C après la mise en contact.

7. Utilisation selon la revendication 6, dans laquelle le marqueur intracellulaire est cCD3, CD79a, cLambda, cKappa, TdT, BCL-2 ou Zap-70.

8. Utilisation selon la revendication 6 ou la revendication 7, pour permettre l'analyse du marqueur intracellulaire pendant au moins 7 jours après la prise de sang.

9. Procédé selon l'une quelconque des revendications 1 à 5, dans lequel l'analyse comprend un dépistage du cancer.

10. Procédé selon l'une quelconque des revendications 1 à 5 ou 9, comprenant en outre une analyse de l'échantillon de sang portant sur un épitope protéique d'un marqueur de surface cellulaire.

11. Procédé selon la revendication 10, dans lequel le marqueur de surface cellulaire est CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, CD200, FMC7, HLA-DR, Kappa, Lambda, TCR αβ, ou TCR γδ.

12. Utilisation selon l'une quelconque des revendications 6 à 8, dans laquelle l'analyse comprend un dépistage du cancer.

13. Utilisation selon l'une quelconque des revendications 6 à 8 ou 12, comprenant en outre une analyse de l'échantillon de sang portant sur un épitope protéique d'un marqueur de surface cellulaire.

14. Utilisation selon la revendication 13, dans laquelle le marqueur de surface cellulaire est CD1a, CD2, CD3, CD4, CD5, CD7, CD8, CD10, CD11b, CD11c, CD13, CD14, CD15, CD16, CD16+56, CD19, CD20, CD22, CD23, CD25, CD26, CD30, CD33, CD34, CD38, CD41, CD45, CD56, CD57, CD58, CD61, CD64, CD71, CD103, CD117, CD123, CD138, CD200, FMC7, HLA-DR, Kappa, Lambda, TCR αβ, ou TCR γδ.
